# EUROPEAN PATENT SPECIFICATION

(11) **EP 0 583 821 B1**
(45) Date of publication and mention of the grant of the patent: **29.03.2000**
(21) Application number: 93202327.8
(22) Date of filing: 22.01.1988
(51) Int. Cl.: A61K 31/495

(54) **Hydralazine as topical treatment for glaucoma**
Hydralazin zur topischen Behandlung von Glaukom
L' hydralazine pour le traitement topique du glaucome

(30) Priority: 23.01.1987 US 6405
(43) Date of publication of application: 23.02.1994
(62) Divisional of application: 88901976.6
(73) Proprietor: THE GENERAL HOSPITAL CORPORATION, Boston, MA 02110 (US)
(72) Inventor: Nathanson, James A., Wellesley, Massachusetts 02181 (US)
(74) Representative: White, Martin Paul

(56) References cited:
- EP-A- 0 227 531
- EP-A- 0 231 752
- ATARASHII GANKA vol. 2, no. 4 , 1985 pages 580 - 582 T. TOMIUGA ET AL. 'Effects of organic nitrates on intraocular pressure in rabbits'
- ANN. OTTALMOL. CLIN. OCUL. vol. 112, no. 5 , 1986 pages 361 - 367 F. FOCOSI ET AL. 'Glyceryl trinitrate and intraocular pressure: a tonographic study in man'
- KLIN. MONATSBL. AUGENHEILKD. vol. 177, no. 3 , 1980 pages 292 - 295 A. & V. WIZEMANN 'Untersuchungen zur ambulanten und perioperativen Augeninnendrucksenkung mit organischen Nitraten.'
- CHEMICAL ABSTRACTS, vol. 76, no. 1, 3 January 1972, Columbus, Ohio, US; abstract no. 110e, V.N. KOSTYUCHENKOV ET AL. 'Effect of adreno- and sympatholytic agents on ophthalmotonus' page 11 ;column 2 ; & AKTUAL. PROBL. FARMAKOL. FARM., VSES. NAUCH. KONF. 1971 pages 155 - 159
- ACTA SOC. OPHTHALM. JPN. vol. 90, no. 10 , 1986 pages 1232 - 1234 T. SASAKI ET AL. 'alpha-Human Atrial Natriuretic Polypeptide (alpha-hANP)'
- M. WINDHOLZ ET AL. 'The Merck Index' 1983 , MERCK & CO., INC. , RAHWAY, N.J., USA * no. 8486: Sodium Nitrite *
- DR. E. MUTSCHLER 'Arzneimittelwirkungen' 1986 , WISSENSCHAFTL. VERLAGSGES. MBH , STUTTGART * page 461 - page 462 *
- J. PHARMACOL. EXP. THER. vol. 260, no. 3 , March 1992 pages 956 - 965 J.A. NATHANSON 'Nitrovasodilators as a new class of ocular hypotensive agents'
- Kostyuchenkov et al.; Aktual. Probl. Farmakol. Farm..; Vses Nauch. Konf. 1971; pages 155-159

## Description

The present invention relates to medicaments which can be used for treating glaucoma.

Glaucoma is a condition of the eye associated with high pressure due to an impediment in the outflow of the aqueous fluid (aqueous humor), which is normally secreted by the ciliary process (a tissue similar in function to the choroid plexus). In 90% of cases the cause is unknown, while in 5%, the condition is secondary to some disease process that blocks the outflow channels. Glaucoma occurs in 2% of all patients over 40; it may be asymptomatic and unrecognized before it progresses to rapid vision loss. The normal pressure is about 15 mmHg. Pressures of 20-30 mmHg may damage the optic nerve and lead to blindness.

Some advances in treating glaucoma have been made with the introduction of beta-adrenergic blockers. However, these agents can have serious pulmonary and cardiovascular side effects, including asthma and congestive heart failure.

According to the present invention there is provided the use of hydralazine in the preparation of a medicament for treating glaucoma; wherein the medicament is adapted for topical administration.

Kostyuchenkov *et al* (*Aktual. Probl. Farmakol. Farm., Vses. Nauch. Konf*. 1971, pages 155-9 (Russ)) disclose that large doses of hydralazine when injected intravenously reduce elastonometric pressures.

EP-A-0227531 is also part of the state of the art, but by virtue of Article 54(3) and Article 54(4) EPC. It discloses various pyroglutamic acid esters, which are said to be useful as dermal penetration enhancers of topically administered antihypertensive drugs such as hydralazine.

Hydralazine is a nitrogen-containing guanylate cyclase activating agent which stimulates cyclic GMP formation in brain and eye secretory tissues. When administered topically, i.e., as eye drops, at very low concentrations, hydralazine causes a substantial decrease in intraocular pressure without observable side effects on the eye. In addition, the topical administration decreases IOP without decreasing systolic, diastolic, or mean systemic blood pressure and without affecting cardiac pulse rate.

Preferably hydralazine is present in solution in a topical medicament at a level of 0.01 to 2.0% (g/100ml).

Hydralazine may be administered as part of a synergistic composition i.e. it may be administered with another compound (e.g. an atriopeptin or atriopeptin agonist, a phosphodiesterase inhibitor or another nitrogen-containing guanylate cyclase activator) so that the correlated action of both compounds has a greater total effect that the sun of this individual effects.

### Description of the Drawings

**Figure 1** (comparative) demonstrates the effect of the administration of nitroglycerine as an eye drop preparation on the IOP in rabbits. The decrease in IOP is not accompanied by a concomitant decrease in systemic blood pressure.
**Figure 2** (comparative) additionally shows the absence of effect on pulse rate or systemic blood pressure from the administration of an eye drop preparation of nitroglycerine in rabbits.
**Figure 3** (comparative) illustrates that doses of nitroglycerine eye drops (50µl of 0.1% aqueous solution) caused no additional decrease in IOP and that lower doses were less effective.
**Figure 4** (comparative) illustrates the effect of the administration of a nitroglycerine eye drop preparation (50µl of 0.1% aqueous solution in one eye) in a volunteer human. Importantly, although the nitroglycerine eye drops decreased IOP, they did not affect systemic blood pressure.
**Figure 5** (comparative) demonstrates that a 50µl 0.1% aqueous eye drop solution of minoxidil, a nitrogen-containing guanylate cyclase activator, decreases the IOP in rabbits.
**Figure 6** (comparative) illustrates that a 50µl 0.1% aqueous eye drop solution of sodium nitrate, another nitrogen-containing guanylate cyclase activator, lowers the IOP in rabbits.
**Figure 7** demonstrates that a 0.1% eye drop preparation of hydralazine decreases the IOP in rabbits.

Having now generally described the invention, the same may be further understood by reference to Example 4 of the following examples. (Examples 1 to 3 are provided for illustrative purposes.)

### EXAMPLE 1 (comparative)

The measurement of intraocular pressure (IOP) was performed using procedures similar to those described in Nathanson, *Brit. J. Pharmacol.* **73**:97 (1981) and Nathanson, *Current Eye Res*. **4**:191 (1985). Briefly, male (3-5 kg), New Zealand white rabbits were housed under standard conditions and exposed to a 12 hr light-dark cycle. IOP was measured with a Perkins applanation tonometer after topical anesthesia with 0.4% benoxinate (and 0.25% sodium fluorescein). The tonometer had been calibrated previously by connecting the anterior chamber of enucleated rabbit eyes to a manometer (and reservoir) and taking tonometer readings at different pressures. A number of preliminary IOP readings were made in order to accommodate the animals to the measurement procedure. Readings were then taken just prior to drug or vehicle injection and at 3, 9, 20, 27, 44, 51, 72, and 144 hours post-injection.

Following topical anesthesia with 0.4% benoxinate, rabbits received rat atrial natriuretic peptide 1-28 (rANP) via a 10 microliter, intravitreal injection, through a 30g ½" needle, the tip of which was positioned at the approximate center of the eye. Drug was dissolved in a solution of artificial aqueous humor containing (in mmol/liter): NaCl 130, KCl 2.7, NaH₂CO₃ 18.3, MgCl₂ 1.33, CaCl₂ 1.5, Glucose 10, HEPES 20, and L-ascorbic acid, 2.5. The contralateral eye received a 10 microliter injection of vehicle alone. In order to evaluate the possible effects of intravitreal injection, *per se*, on IOP, additional rabbits received a 10 microliter intravitreal injection of vehicle in one eye only.

### EXAMPLE 2 (comparative)

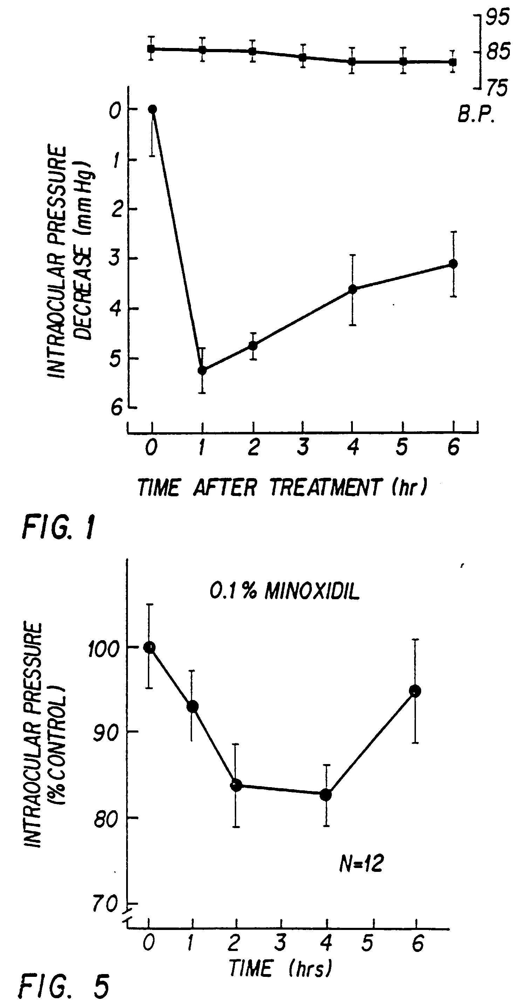

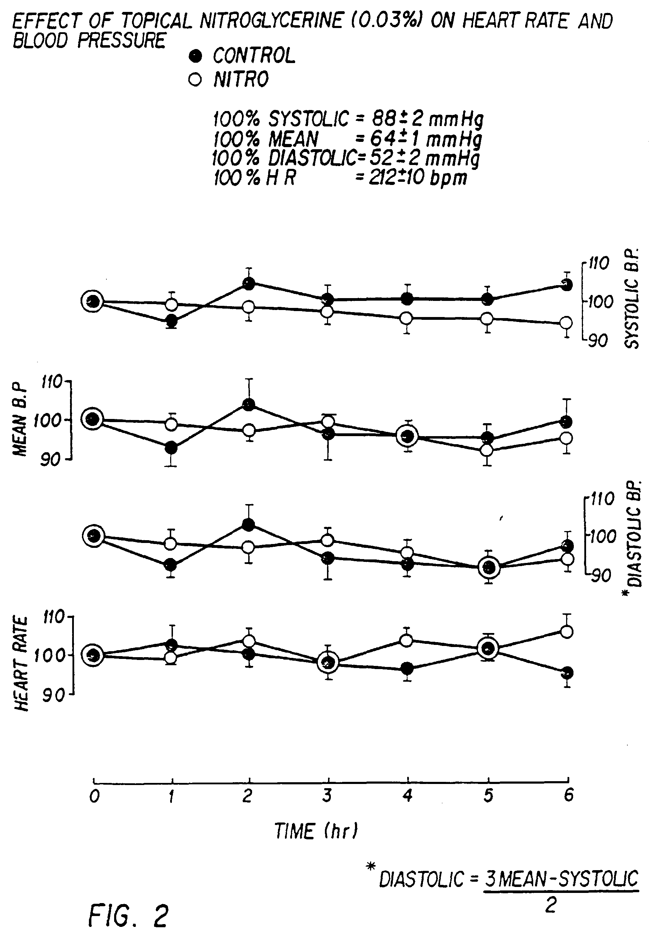

The effect of nitroglycerine eye drops on rabbit intraocular pressure was also measured. The IOP of 8 conscious, male albino rabbits (3-4 kg) was measured by applanation pnumatonometry after topical anesthesia with 0.4% benoxinate. Nitroglycerine in phosphate buffered saline, pH 7.4, was given unilaterally, in two 25µl drops spaced five minutes apart. Blood pressure and pulse rate were measured by a photoelectric detector using an automated external tail cuff. Figure 1 shows the results of the topical administration of a 0.03% (gm/100ml) nitroglycerine solution on IOP. A marked decrease in IOP (5.2mm Hg at maximum), which was greatest at one hour and still significantly reduced at six hours, can be seen. By 24 hours, pressure had returned to normal. Importantly, there was no significant change in pulse rate or in systolic, diastolic or mean blood pressure, as can also be seen in Figure 2.

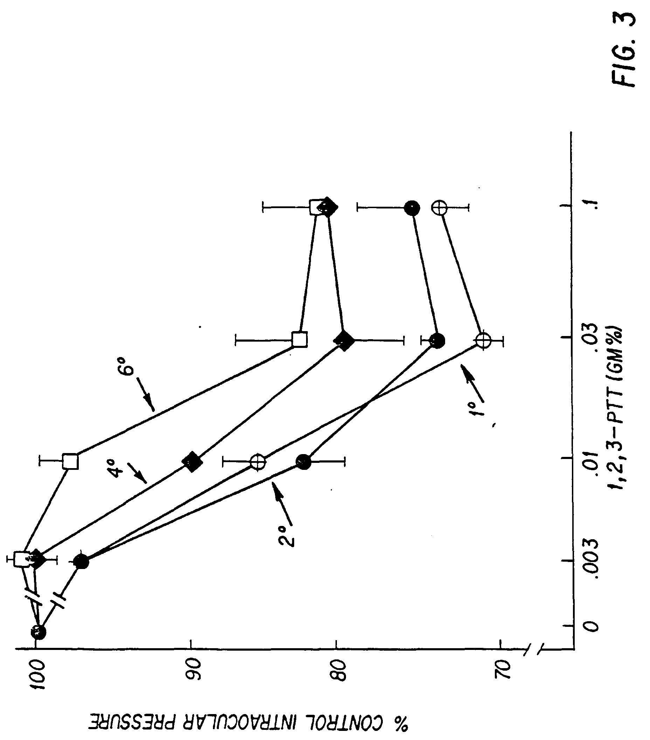

As Figure 3 illustrates, higher doses of nitroglycerine caused no additional decrease in IOP, and lower doses were less effective. Each curve demonstrates the ± mean of eight rabbits at various times after administration of 50µl eye drops containing nitroglycerine in neutral aqueous solution at the dose shown. At higher doses, contralateral eyes also showed a decrease in pressure, although significantly less, ranging from 40-75% of that seen ipsilaterally. At no dose did conjunctival inflammation nor any change in pupillary diameter occur. At higher doses (0.1%), there was a slight, transient (five minutes) hyperemia of the conjunctival vessels.

This experiment indicates that direct topical ocular administration of a guanylate cyclase activator as an eye drop can decrease IOP without any significant change in systemic cardiovascular parameters. Thus, administration of nitroglycerine as an eye drop involves a more selective delivery to end organ tissues in the eye.

### EXAMPLE 3 (comparative)

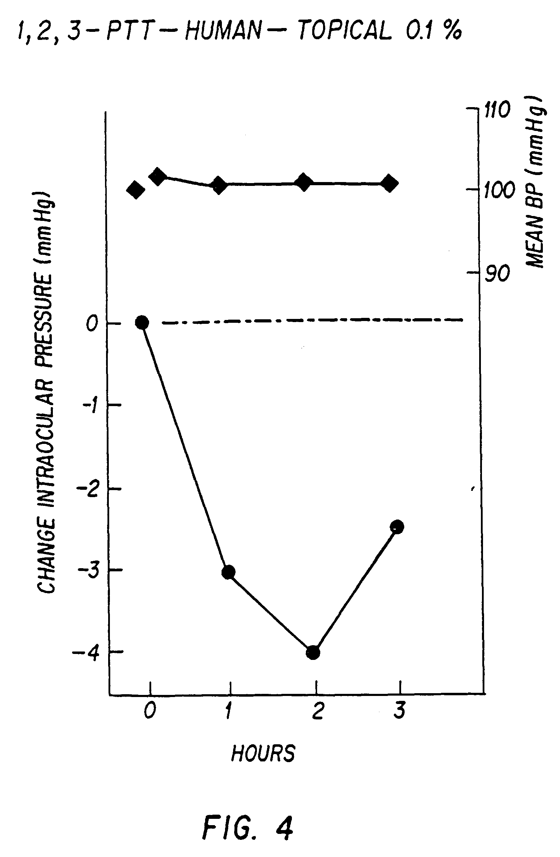

The effect of administration of a nitroglycerine eye drop preparation (50µl of 0.1% aqueous solution in one eye) in a volunteer human is illustrated in Figure 4. IOP was measured by applanation pnumatonometry and blood pressure and pulse were measured by arm cuff. Topical administration of nitroglycerine significantly decreased IOP by formula mmHg without change in systemic blood pressure (shown) or pulse (not shown).

### EXAMPLE 4

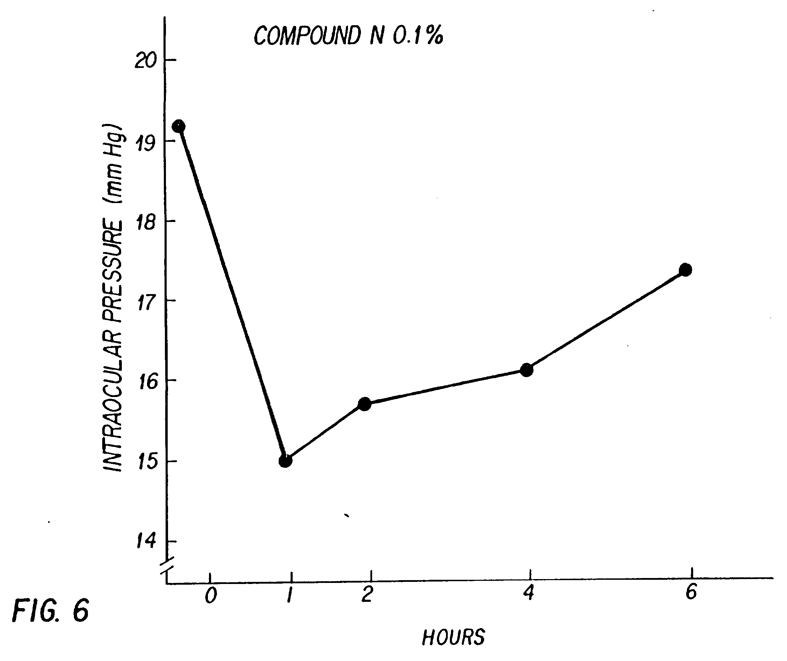

As Table 2 (comparative) indicates, minoxidil (Compound "M") also increases guanylate cyclase activity alone, and to an even greater extent when combined with rANP. Figure 5 illustrates the effect of a 50µl of 0.1% aqueous solution of minoxidil on intraocular pressure in 12 rabbits. Minoxidil caused a significant decrease in IOP.

**TABLE 2 (comparative)**

| CILIARY PROCESS GUANYLATE CYCLASE ACTIVITY (Percent Increase) | |
|---|---|
| COMPOUND "M" (10⁻⁴M) | 14 ± 4 |
| rANP 1-28 (10⁻⁶M) | 60 ± 27 |
| COMPOUND "M" + rANP | 210 ± 22 |

As Table 3 (comparative) indicates, another compound, sodium nitrate (Compound "N") was found to increase ciliary process guanylate cyclase activity and Figure 6 illustrates the effect of sodium nitrate given as 50µl in a 0.1% eye drop preparation. Experimental procedures were similar to those described in Example 2. Sodium nitrate lowers IOP in rabbits.

**TABLE 3 (comparative)**

| CILIARY PROCESS GUANYLATE CYCLASE ACTIVITY (Percent Increase) | |
|---|---|
| COMPOUND "N" | 46 |
| rANP 1-28 | 74 |

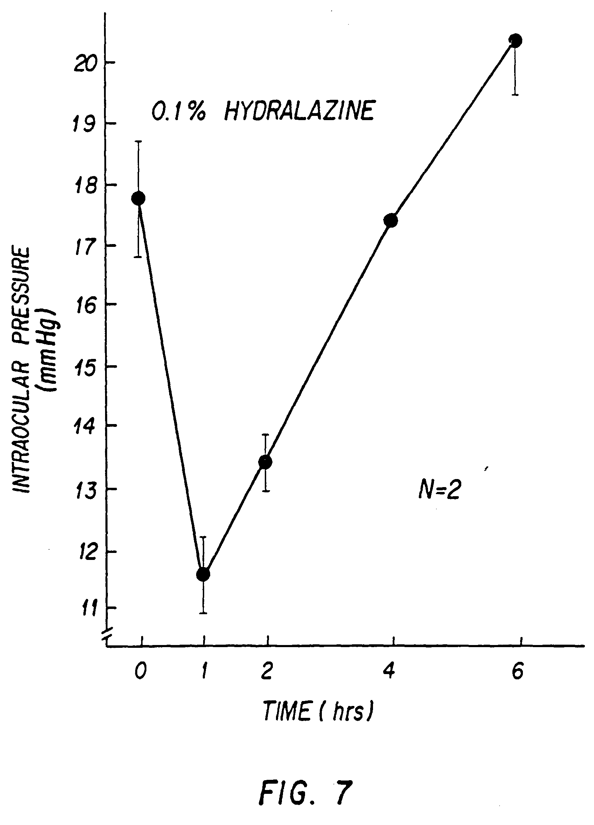

Finally, hydralazine, a drug thought to work through activation of guanylate cyclase activation, when given to rabbits as a 0.1% eye drop preparation in normal saline, decreases IOP. Experimental procedures used were similar to those described in Example 2. Figure 7 illustrates this effect.

## Claims (Claims for the following Contracting State(s): CH, DE, FR, GB, IT, LI, NL)

1. The use of hydralazine in the preparation of a medicament for treating glaucoma; wherein the medicament is adapted for topical administration.

2. The use according to claim 1; wherein the medicament is in the form of eye drops.

3. The use according to claim 2; wherein the hydralazine is present in solution in the medicament at a level of 0.01 to 2.0% (g/100ml).

4. A medicament comprising hydralazine, which is adapted for topical administration; wherein said medicament does not comprise a pyroglutamic acid ester dermal absorption enhancing agent of the formula: 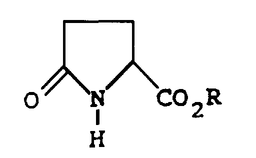 , in which R is a straight or branched chain alkyl (C₅-C₂₀), alkenyl (C₅-C₂₀) with 1-6 double bonds, hydroxyalkyl (C₅-C₂₀) with 1-3 hydroxy groups, ketoalkyl (C₅-C₂₀), unsaturated hydroxyalkyl (C₅-C₂₀) carboxyalkyl (C₅-C₂₀) or alkoxycarbonylalkyl (C₅-C₂₀).

5. A medicament comprising hydralazine, which is adapted for topical administration and which is in the form of eye drops.

6. A medicament according to claim 5; wherein the hydralazine is present in solution at a level of 0.01% to 2.0% (g/100ml).

## Claims (Claims for the following Contracting State(s): AT, BE, LU, SE)

1. The use of hydralazine in the preparation of a medicament for treating glaucoma; wherein the medicament is adapted for topical administration.

2. The use according to claim 1; wherein the medicament is in the form of eye drops.

3. The use according to claim 2; wherein the hydralazine is present in solution in the medicament at a level of 0.01 to 2.0% (g/100ml).

4. A medicament comprising hydralazine, which is adapted for topical administration.

5. A medicament according to claim 4, which is in the form of eye drops.

6. A medicament according to claim 5; wherein the hydralazine is present in solution at a level of 0.01% to 2.0% (g/100ml).

## Patentansprüche (Patentansprüche für folgende(n) Vertragsstaat(en): CH, DE, FR, GB, IT, LI, NL)

1. Verwendung von Hydralazin bei der Herstellung eines Medikaments zur Behandlung von Glaucom, wobei das Medikament für eine topische Anwendung adaptiert ist.

2. Verwendung nach Anspruch 1, wobei das Medikament in Form von Augentropfen vorliegt.

3. Verwendung nach Anspruch 2, wobei das Hydralazin im Medikament in Lösung in einer Menge von 0,01 bis 2,0 % (g/100 ml) vorliegt.

4. Ein Hydralazin enthaltendes Medikament, welches für die topische Anwendung adaptiert ist, wobei das Medikament eine die Hautabsorption von Pyroglutaminsäure verstärkende Verbindung der Formel 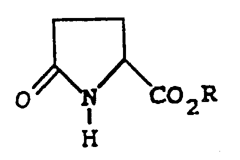 nicht enthält, in welcher R eine gerade oder verzweigte Kette von Alkyl (C₅ - C₂₀), Alkenyl (C₅ - C₂₀) mit 1 - 6 Doppelbindungen, Hydroxyalkyl (C₅ - C₂₀) mit 1 - 3 Hydroxylgruppen, Ketoalkyl (C₅ - C₂₀), ungesättigtes Hydroxyalkyl (C₅ - C₂₀), Carboxyalkyl (C₅ - C₂₀) oder Alkoxycarbonylalkyl (C₅ - C₂₀) bedeutet.

5. Medikament nach Anspruch 4, welches für die topische Anwendung adaptiert ist und in Form von Augentropfen vorliegt.

6. Medikament nach Anspruch 5, in welchem Hydralazin in Lösung in einer Menge von 0,01 % bis 2,0 % (g/100 ml) vorliegt.

## Patentansprüche (Patentansprüche für folgende(n) Vertragsstaat(en): AT, BE, LU, SE)

1. Verwendung von Hydralazin bei der Herstellung eines Medikaments zur Behandlung von Glaucom, wobei das Medikament für eine topische Anwendung adaptiert ist.

2. Verwendung nach Anspruch 1, wobei das Medikament in Form von Augentropfen vorliegt.

3. Verwendung nach Anspruch 2, wobei das Hydralazin im Medikament in Lösung in einer Menge von 0,01 bis 2,0 % (g/100 ml) vorliegt.

4. Ein Hydralazin enthaltendes Medikament, welches für topische Anwendung adaptiert ist.

5. Medikament nach Anspruch 4, welches in Form von Augentropfen vorliegt.

6. Medikament nach Anspruch 5, in welchem Hydralazin in Lösung in einer Menge von 0,01 % bis 2,0 % (g/100 ml) vorliegt.

## Revendications (Revendications pour l'(les) Etat(s) contractant(s) suivant(s): CH, DE, FR, GB, IT, LI, NL)

1. Utilisation de l'hydralazine dans la préparation d'un médicament pour le traitement du glaucome, dans laquelle le médicament est adapté pour l'administration topique.

2. Utilisation selon la revendication 1, dans laquelle le médicament est sous la forme de gouttes ophtalmiques.

3. Utilisation selon la revendication 2, dans laquelle l'hydralazine est présente en solution dans le médicament à un taux de 0,01 à 2,0% (g/100 ml).

4. Médicament comprenant de l'hydralazine qui est adapté pour l'administration topique, dans lequel ledit médicament ne comprend pas un agent augmentant l'absorption dermique d'ester de l'acide pyroglutamique de formule 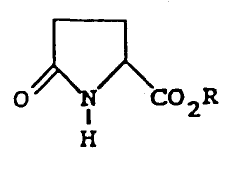 dans laquelle R est une chaîne d'alkyle en (C₅-C₂₀) linéaire ou ramifiée, alcényle en (C₅-C₂₀) avec 1-6 doubles liaisons, hydroxyalkyle en (C₅-C₂₀) avec 1-3 groupements hydroxy, cétoalkyle en (C₅-C₂₀), hydroxyalkyle insaturé en (C₅-C₂₀), carboxyalkyle en (C₅-C₂₀) ou alkoxycarbonylalkyle en (C₅-C₂₀).

5. Médicament comprenant de l'hydralazine, qui est adapté pour l'administration topique et qui est sous la forme de gouttes ophtalmiques.

6. Médicament selon la revendication 5, dans lequel l'hydralazine est présente en solution à un taux de 0,01% à 2,0% (g/100 ml).

## Revendications (Revendications pour l'(les) Etat(s) contractant(s) suivant(s): AT, BE, LU, SE)

1. Utilisation de l'hydralazine dans la préparation d'un médicament pour le traitement du glaucome, dans laquelle le médicament est adapté pour l'administration topique.

2. Utilisation selon la revendication 1, dans laquelle le médicament est sous la forme de gouttes ophtalmiques.

3. Utilisation selon la revendication 2, dans laquelle l'hydralazine est présente en solution dans le médicament à un taux de 0,01 à 2,0% (g/100 ml).

4. Médicament comprenant de l'hydralazine, qui est adapté pour l'administration topique.

5. Médicament selon la revendication 4, qui est sous la forme de gouttes ophtalmiques.

6. Médicament selon la revendication 5, dans lequel l'hydralazine est présente en solution à un taux de 0,01% à 2,0% (g/100 ml).
